# EUROPEAN PATENT APPLICATION

(11) **EP 1 475 437 A1**
(43) Date of publication of application: **10.11.2004**
(21) Application number: 03777273.8
(22) Date of filing: 04.12.2003
(51) Int. Cl.: C12N 15/09, C12N 15/63

(54) **METHOD OF EFFICIENTLY RECOMBINING ORF ENCODED BY cDNA AND TEMPLATE VECTOR, TRAP VECTOR AND PRIMER TO BE USED THEREIN**

(30) Priority: 10.12.2002 JP 2002357566
(71) Applicant: Kazusa DNA Research Institute Foundation, Kisarazu-shi, Chiba 292-0812 (JP)
(72) Inventor: NAGASE, Takahiro, KAZUSA DNA RESEARCH INST. FOUND., Kisarazu-shi, Chiba 292-0812 (JP); NAKAJIMA, Daisuke, KAZUSA DNA RESEARCH INST. FOUND, Kisarazushi, Chiba 292-0812 (JP); OHARA, Osamu, c/o KAZUSA DNA RESEARCH INST. FOUND., Kisarazu-shi, Chiba 292-0812 (JP)
(74) Representative: Woods, Geoffrey Corlett
(86) International application number: PCT/JP2003/015571
(87) International publication number: WO 2004/053123

(57) **Abstract**

The object of the present invention is to solve the problems in excising a DNA region (or gene) with conventional restriction enzymes, or in amplification of the DNA region by PCR.

The present invention provides a method for precisely, quickly and simply cloning a target ORF, for example the target ORF encoding a protein, particularly the target ORF encoding a long-chain ORF comprising bases as many as several thousand bp in cDNA, and means such as various vectors used for the cloning method.

## Description

### Technical Field

The present Invention relates to a method of efficient recombination of open reading frames (ORF) encoded in cDNA. and a template vector, trap vector and primer used thereto.

### Background Art

Gene cloning is an essential technique in gene research as a means for separating and amplifying a specified gene or DNA. While human genome draft sequences have been published since February, 2001, by large-scale sequencing in the human genome projet, accurate cloning of genes composing base sequences having as many as several thousand base pairs (bp) has become a crucial problem today in studies of analyzing the functions of genes contained in the genome, which may be referred to as "functional genomics".

in usual gene cloning, a fragment containing a desired gene is excised with an appropriate restriction enzyme, the excised fragment is inserted into an appropriate cloning vector such as a plasmid vector, cosmid vector derived from a λ-bacteriophage, bacteriophage P1 vector, bacteria-derived artificial chromosome (BAC) or P1-derived artificial chromosome (PAC) excised with the same restriction enzyme to produce a recombinant DNA molecule (a recombinant vector), and the recombinant DNA molecule is amplified by taking advantage of a host such as a bacteria, e.g Escherichia coil, or a phage to obtain a large quantity of the recombinant DNA molecules.

However, restriction enzyme sites need to be appropriately located at appropriate positions In this method to properly excise a fragment containing the gene which is the object of cloning (target DNA region). Moreover, the fragment excised with the restriction enzyme need to be purified before it is inserted into the cloning vector. Accordingly, it is difficult to clone a gene comprising a base sequence as large as several, thousand bp.

Alternatively, obtaining the DNA sequence of the target gene In a large quantity has become possible by a method called polymerase chain reaction (PCR), which is an in vitro reaction using a primer designed based on a known amino acid sequence or base sequence by taking advantage of DNA synthesis with a DNAsynthetase.

However, since it has been estimated that misreading occurs once per 400 to 5,000 bases in DNA synthesis using the DNA synthetase in PCR, the accuracy and reproducibility of the gene sequence obtained by amplification may not be so reliable.

Novel gene cloning methods based on a homologous recombination principle described in the following non-patent documents 1 and 2 have been proposed for solving the problems described above.

Non-patent Document 1: Youming Zhang et. al., NATURE BIOCHEMISTRY, Vol. 18, "DNA Cloning by Homologous Recombination in Escherichia coli", 2000, pp1314-1317

Non-patent Document 2: Joep P. P. Muyrers et al., TRENDS In Biochemical Sciences, Vol. 26, "Techniques: Recombinogenic Engineering - Options for Cloning and Manipulating DNA", pp325-331

Accordingly, an object of the present invention is to solve the problems above in excising DNA regions (or genes) using conventional restriction enzymes, or in amplification of the DNA regions by PCR. Another object of the present invention is to provide a method for accurately, quickly and simply cloning a target ORF (gene), for example a protein-encoding target ORF, particularly a long-chain ORF comprising as many as several thousand bp, by taking advantage of homologous recombination reactions, and means such as various vectors used thereto.

### Disclosure of Invention

In a first aspect, the invention provides a template vector for a trap vector comprising a replication starting point, a first drug tolerance gene, a first primer-binding sequence, a second primer-binding sequence, and a suicide gene inserted between the first and second primer-binding sequences.

In a second aspect, the invention provides PCR primers for forming a trap vector comprising a first primer comprising an antisense sequence of the 5'-end sequence of a target open reading frame (ORF) and an antisense sequence of the first primer-binding sequence linked to the 3'-side thereof, and a second primer comprising a sense sequence of the 3'-end sequence of the target ORF and a sense sequence of the second primer-binding sequence linked to the 3'-side thereof.

In a third aspect, the invention provides a kit for forming a trap vector comprising the template vector for the trap vector and the PCR primer described above, and a method for forming a linear trap vector by PCR using the kit for forming the trap vector.

In a fourth aspect, the invention provides a method for forming a cloning vector containing the target ORF in the trap vector by a homologous recombination reaction between the trap vector and the target ORF.

In a fifth aspect, the invention provides a cloning vector obtained by the cloning method described above.

In a sixth aspect, the invention provides a cloning method of the target ORF. comprising a step of amplifying or proliferating the cloning vector.

In a seventh aspect, the Invention provides the gene itself encoded in the target ORF cloned as described above.

### Brief Description of the Drawings

Fig. 1 shows a structure of the PCR product comprising an attB site.

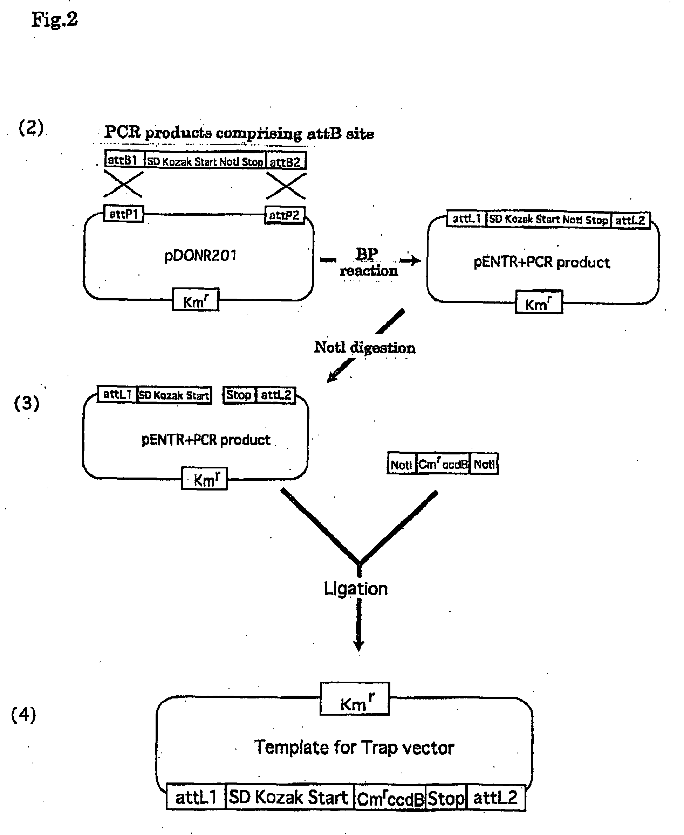

Fig. 2 shows a scheme of each step for producing a cloning vector containing the target ORF taking advantage of homologous recombination of the present invention.
Fig. 3 is the continuation of Fig. 2, and shows a scheme of each step for producing a cloning vector containing the target ORF taking advantage of homologous recombination of the present invention.
Fig. 4 is a photograph showing the result of electrophoresis to confirm an in vitro transcription/translation reaction product (a band at about 210 kDa) of pT7DESThg 00295N1.
Fig. 5 shows a photograph obtained by an Western blotting analysis of a protein obtained from culture cells expressing the clone obtained by an ORFT trap method of the present invention using an anti-GFP antibody.

### Best Mode for Carrying Out the Invention

The "replication origin" In the template vector for a trap vector in the first aspect of the present invention is an essential sequence for replicating the template vector and the trap vector generated from the template vector in a host. Any sequences well known to those skilled In the art may be selected depending on the vectors used. A "drug tolerance gene" refers to an arbitrary sequence having a function of enabling the vector containing the gene to be proliferated in an appropriate selection medium, and examples of the gene include tolerance genas against antibiotics such as kanamycin, ampicillin and chloramphenicol.

The "first primer-binding sequence" and "second primer-binding sequence" as used in the specification of the present Invention refer to the sequences which may complementarity bind to the first and second primers, respectively, of the present Invention in order to produce a linear trap vector by PCR. Accordingly, while such sequences are not particularly limited so long as they are able to form complimentary bonds with the primers, the first primer-binding sequence comprising at least a part of a Kozak consensus sequence containing a translation start codon of the eukaryotic cells, for example, being preferred so that a finally cloned target ORF can be translated in eukaryotic cells. When the sequence further comprises a Shine-Dalgamo sequence, which is a ribosome binding site In Escherichia coil, at an appropriate site upstream of the 5'-end of the Kozak consensus sequence (for example, at a site 3 to 10 bases apart from the start codon), the ORF region can be translated In Escherichia coli cells a well.

On the other hand, the second primer-binding sequence preferably comprises TAG or TAN (N denotes G, A, T or C). When the second primer-binding sequence containing TGA, TAA or TAG sequences as stop codons is used, a trap vector (native type) that is translated Into only the target ORF as proteins is obtained, while a trap vector (fusion type) that is translated as fusion proteins is obtained when the sequence contains TGT and other sequences since this portion is read-through and an appropriate ORF succeeding thereto is translated. Accordingly, a fusion protein between a protein encoded by the target ORF and an appropriate tag peptide (such as 6 x His, FLAG and HA) is expressed by binding a base sequence that encodes the tag peptide downstream of the target ORF. These proteins may be readily purified by taking advantage of an antibody against the tag peptide or affinity chromatography, or localization of these proteins in culture cells may be analyzed by allowing the protein to be expressed or recognized in the cells. The trap vectors of the native type and fusion type may be simultaneously obtained by synthesizing and using these first and second primer-binding sequences containing the respective sequences described above. The two finally obtained cloning vectors are able to readily identify and isolate the clones of both types by excising the vectors at restriction enzyme sites containing the TGN and TAN sequences, which have been further comprised in the vector. While the restriction enzyme sites are not particularly limited, they are preferably obtained by replacement of 1 to 2 bases of the stop codon. Examples of the restriction sites include Xba1, SreBl, BC1I, Fspl, Mscl, Nrul and BspHl.

While the numbers of base sequences of the first primer-binding sequence and second primer-binding sequence are not particularly limited so long as binding to the primers is specific, the sequences comprise at least five bases, more preferably 7 to 25 bases, and the first primer-binding sequence preferably comprises at least a part of the Kozak consensus sequence and shine-Dalgarno sequence.

The "suicide gene" inserted between the first and second primer-binding sequences is used for inhibiting the proliferation of host cells transformed with the template vector for the trap vector containing the suicide gene, and examples of the suicide gene include arbitrary genes well known to those skilled In the art such as ccdB (Philippe Bernard wt.Al., Gene, Vol., 148, Positive-Selection Vectors Using the F Plasmid ccdB Killer gene, pp71-74).

Preparation of the template vector for the trap vector of the present invention may be facilitated by further inserting a second drug tolerance gene that is different from the first drug tolerance gene between the first and second primer-binding sequences.

The template vector for the trap vector of the present Invention may be readily prepared using a gene engineering method well known to those skilled in the art. Those skilled in the art would be able to prepare the template vector for the trap vector of the present Invention starting from an appropriate Escherichia coli plasmid vector or a vector derived from a phage well known to those skilled, in the art. Alternatively, the vector for the trap vector of the present invention may be prepared using restriction enzymes, PCR or Gateway (trade name) technology.

For example, the template vector for the trap vector of the present invention can be prepared using a BP reaction in a site-specific recombination system of λ-phage as shown in the following examples.

The BP reaction in the site-specific recombination system of λ-phage is performed between an appropriate starting vector comprising the replication origin, first drug tolerance gene and attP1 and attB2 regions, and a sequence that functions as the first and second primer-binding sequences between attB1 and attb2 regions (for example, at least a part of the Kozak consensus sequence, at least a part of the Shine-Dalgarno sequence, or a base sequence fragment containing TGN or TAN), and then the second drug tolerance gene and suicide gene are inserted between attL1 and attL2 by restriction enzyme treatment. Consequently, the template vector for the trap vector comprising the first and second primer-binding sequences between attL1 and attL2 are prepared. The base sequence fragment for the above sequences may be prepared by a method known to those skilled in the art such as PCR.

As PCR primers for preparing the trap vector of the present invention are used the first primer comprising an antisense sequence of the 5'-end sequence of the target ORF and an antisense sequence of the first primer-binding sequence linked to the 3'-side thereof, and the second primer comprising a sense sequence of the 3'-end sequence of the target ORF and a sense sequence of the second primer-binding sequence linked to the 3-side thereof. As long as a significant homologous recombination reaction occurs between the trap vector obtained by PCR and the target ORF so that the target ORF may be accurately and efficiently inserted In the trap vector, the numbers of bases in the 5'-end sequence and 3'-end sequence of the target ORF are not particularly limited. The 5'-end sequence and 3'-end sequence preferably comprise at least 10 bases, more preferably 25 to 60 bases.

The linear trap vector can be prepared using a kit containing the template vector as a template for the trap vector and the PCR primer. PCR itself is well known In the art, and may be readily carried out by those skilled in the art by appropriately selecting PCR conditions. The kit may contain buffer solutions, polymerase and other reagents necessary for PCR.

Since the linear trap vector thus formed have the 5'-end sequence of the target ORF and 3'-end sequence of the target ORF at its each end, respectively, only the target ORF Is Inserted into the trap vector by a homologous recombination reaction between the trap vector and the target ORF to enable the cloning vector to be produced. Untranslated regions (UTR) are present upstream and downstream of the ORF region in usual expression systems, and expression of proteins is blocked by various influences of these UTRs when the ORF region is translated into the proteins. On the contrary, it is possible to eliminate unnecessary untranslated regions (UTR) upstream and downstream of the ORF contained in the cloning vector prepared in the present invention, and the problems above may be avoided when the proteins are expressed in each expression system.

The target ORF may be provided in various forms. For example, the target ORF may be a part of a genome, or may be provided as cDNA inserted into a cosmid vector derived from λ-bacteriophage, bacteriophage P1 vector, artificial chromosome of bacteria, or P1 derived artificial chromosome. Alternatively, the target ORF may be a part of a synthetic DNA.

The target ORF has a high possibility of containing a gene encoding genetic information of proteins. The preferable target ORF according to the present invention Is cDNA contained in the vector, and a target ORF as a long-chain cDNA comprising a base sequence as long as several thousand bp or more up to ten and several thousands bp may be advantageously cloned.

Homologous recombination itself is well known to those skilled in the art. For example, bacteria are co-transformed with the trap vector and a vector comprising the ORF region using an appropriate method well known to those skilled in the art such as electroporation, and a homologous recombination reaction is carried out in the bacteria. Since linear DNA fragments may be digested by a strong exonuclease activity when RecBCD is used, the homologous recombination is preferably performed using RecE and RecT enzymes.

A regeneration colony may be obtained by cultivating the bacteria thus transformed at an appropriate temperature and within an appropriate time, for example, for 1 to several hours at 37°C. A part of the regeneration colony thus obtained is cultivated in an appropriate culture medium containing a drug corresponding to the first drug tolerance gene, and the target ORF can be cloned by proliferating the cloning vector-containing bacteria of the present invention. A larger number of colonies may be obtained by collecting the bacteria at a turbidity (about 0.3 to 0.4 at 600 nm) lower than the usual level (about 0.5 to 0.6) when competent cells used for co-transformation above are prepared.

Accordingly, bacteria such as Escherichia coli having a high homologous recombination activity are preferably used for preparing the cloning vector comprising the target ORF by the homologous recombination reaction. Examples of Escherichia coli having such high homologous recombination activity include the RecE/RecT-expressing sbcA strain (Clark, A. J. et. al., Genes of the RecE and RecF pathways of conjugation/recombination in Escherichia coil, Cold Spring Herb. Symp. Quant. Biol., 49, 453-462; Hall, S. D., Kane, M. F. & Kolodner, R. D., identification and characterization of the Escherichia coli RecT protein encoded by the RecE region that promotes renaturation of homologous single-stranded DNA, J. Bacteriol., 175, 277-287), particularly JC8679 and JC9604. It is also possible to use a transformant of an appropriate Escherichia coli strain with a plasmid having the RecE/RecT gene. Several examples of Escherichia coil having a high homologous recombination activity obtained by transformation as described above are described in non-patent documents 1 and 2.

The target ORF can be cloned by amplifying the cloning vector of the present invention in an appropriate host system. Accordingly, it is not required in the cloning method of the present invention to previously purify and isolate DNAs containing the target ORF, which is different from conventional methods in which DNAs are cloned using restriction enzymes. Since the target ORF is amplified by taking advantage of replication mechanisms in the Escherichia coil, mutation of base sequences such as that by Taq polymerase observed in conventional amplification methods using PCR hardly occurs.

When the template vector for the trap vector comprising the first and second primer-binding sequences between attL1 and attL2, which is prepared using a BP reaction in the site-specific recombination system of λ-phage, Is used, the cloning vector of the present invention Is able to function as an entry clone in Gateway (trade name) technology (cloning technology). In this Gateway technology, expression clones linked to promoters and tags for various desired expression systems may be obtained with high throughput while maintaining the target ORF by a LR reaction, which is a site-specific recombination reaction of λ-phage between the entry clone and various destination vectors.

By previously inserting a transcription regulation sequence such as a promoter Into an appropriate site of the template vector for the trap vector according to the present invention, the cloning vector of the present invention can be immediately used as an expression vector that functions in an expression system corresponding to the promoter although it does not function as the entry clone used in the Gateway technology.

While the present invention is described in more detail hereinafter with reference to examples, the invention is by no means limited to these examples. Genes were manipulated In the examples according to a method well known to those skilled In the art described In "Latest Protocol of Molecular Biology", ed. by Frederick M. Ausubel et al., 1987.

Schemes (1) to (7) In each step described in the following examples are shown In the drawings.

### Example 1

### (1) Preparation of a PCR product comprising the attB site

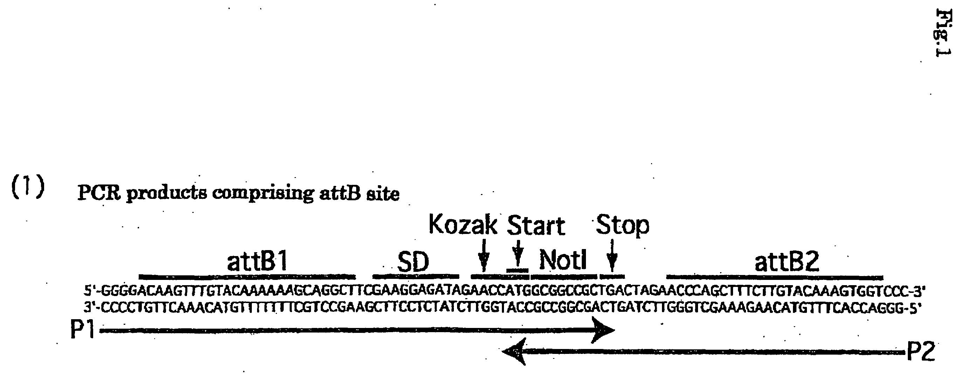

PCR was performed in a 50-µl volume (5 µl of dNTP (2.6 mM each), 5 µl of 10 × LA PCR buffer (Mg²⁺ plus), 1 µl of P1 (100 pmol/µl), 1 µl of P2 (100 pmol/µl), 0.6 µl of LA Taq (Takara Bio Inc.), 37.5 µl of DDW). The PCR comprises 10 cycles of denaturation at 94°C for 3 minutes, denaturation at 94°C for 15 seconds, annealing at 30°C for 30 seconds and elongation at 68°C for 15 seconds; followed by 20 cycles of denaturation at 94°C for 15 seconds, annealing at 55°C for 30 seconds and elongation at 68°C for 15 seconds and kept cool at 4°C. The PCR product was stored at 4°C. The PCR solution containing the PCR product comprising the attB site thus obtained (the sequence shown in Fig. 1(1)) was purified using a Concert Rapid PCR purification kit manufactured by Invitrogen Co., and the PCR product was eluted in 50 µl of TE (10 mM Tris (pH 8.0), 0.1 mM EDTA).

### (2) Preparation of pENTR + PCR products

The PCR product comprising the attB site was inserted into a pDONR221 donor vector (manufactured by invitrogen Co.). The reaction was carried out in a 10-µl volume (5 µl of the PCR product comprising the attB site, 1 µl of pDONR202 (150 ng/µl), 2 µl of 5 × BP buffer, 2 µl of BP Clonase Enzyme Mix). After allowing the reaction solution to stand at a room temperature for 1 hour, the solution was mixed with 1 µl of proteinase K and warmed at 37°C for 10 minutes. After mixing 1 µl of the BP reaction solution with 25 µl of competent cells for the DH5α calcium method (trade name LIBRARY EFFICIENCY DH5α, manufactured by Invitrogen Co.), the solution was allowed to stand on ice for 30 minutes. After applying heat shock treatment at 42°C for 30 seconds, the solution was cooled on ice for 2 minutes. The cells were cultivated at 37°C after the addition of 200 µl of SOC, and 10 µl of the cultivation solution was applied on a plate containing 50 µg/ml of kanamycin so that 87 regeneration colonies were obtained. After the colony was seeded on 2-ml of LB liquid culture medium containing 50 µg/ml of kanamycin and cultivated with shaking (37°C, O/N), 75 µl (119 ng/µl) of the pENTR + PCR products were obtained using a Concert Rapid Plasmid purification kit manufactured by Invitrogen Co.

### (3) and (4) Preparation of the template vector for the trap vector

A ccd gene was introduced into the Notl site of the pENTR + PCR products. After digesting the pENTR + PCR products with Notl (37°C, 3 hrs) In a 50-µl volume (43 µl of pENTR + PCR products, 2 µl of Notl (Takara Bio inc.), 5 µl of H-buffer (Takara Bio Inc.)), the product was treated with PCl (phenol/chloroform/isoamyl alcohol) and precipitated with ethanol. The precipitate was dephosphorized (65°C, 30 minutes) in a 100-µl volume (88 µl of a DNA solution, 10 µl of 10 × AP buffer (Takara Bio Inc.), 2 µl of alkaline phosphatase (Takara Bio Inc.)), and the product was dissolved in 30 µl of TE after the PCl treatment. A Cm'ccd fragment having Notl sites added at both ends (an 843-bp to 2617-bp fragment of pDONR201 was amplified with a PCR primer comprising the Notl site) was prepared. These, samples were ligated (room temperature, 3.6 hours) In an 8-µl volume (0.5 µl of Notl digestion product (dephosphorized) of pENTR + PCR products and 2.5 µ of the Cm^{r}ccd fragment having Notl sites added at both ends, and 2 µl of 2 × ligation buffer (pGRMT-E kit manufactured by Promega Co.), 1 µl of T4 DNA ligase (Promega pGRMT-E kit manufactured by Promega Co.)). This ligation solution (1 µl) was mixed with 10 µl of competent calls for the DB 3.1 calcium method (LIBRARY EFFICIENCY DB3:1 Competent Cells manufactured by Invitrogen Co.), and heat shock treatment was applied to the mixed solution at 42°C for 45 seconds after cooling on Ice for 30 minutes, followed by cooling on ice for 2 minutes. After the addition of 100 µl of SOC, the cells were cultivated at 37°C for 30 minutes, and 100 µl of the cultivation medium was applied on a plate containing 50 µg/ml of kanamycin and 25 µg/ml of chloramphenicol. The regenerated colony was cultivated with shaking (37°C, O/N) in 2 ml of LB liquid culture medium containing 50 µg/ml of kanamycin and 25 µg/ml of chloramphenicol, and a plasmid was prepared using a kit manufactured by BRL Co, thereby obtaining 75 µl (367 ng/µl) of the template vector for the trap vector.

### (5) and (6) Preparation of the trap vector

The trap vector for sub-cloning the ORF was prepared from clone hg00295 containing the myosin light chain kinase gene by PCR. PCR was performed in a 50-µl volume (5 µl of dNTP (2.5 mM each), 5 µl of 10 × LA PCR buffer (Mg²⁺ plus), 5 µl of P3 (10 pmol/µl), 5 µl of P4 (10 pmol/µl), 0.5 µl of LA Taq (Takara Blo Inc.) and 24.5 µl of DDW). The PCR comprises 5 cycles of denaturation at 94°C for 5 minutes, denaturation at 94°C for 30 seconds, annealing at 40°C for 30 seconds and elongation at 72°C for 2 minutes, followed by 20 cycles of denaturation at 94°C for 30 seconds, annealing at 55°C for 30 seconds and elongation at 72°C for 2 minutes. The PCR product was stored at 4°C. The PCR solution containing the trap vector was purified using a Concert Rapid PCR purification kit manufactured by Invitrogen Co., and the product was eluted in 50 µl of TE (10 mM- Tris (pH 8.0), 0.1 mM of EDTA).

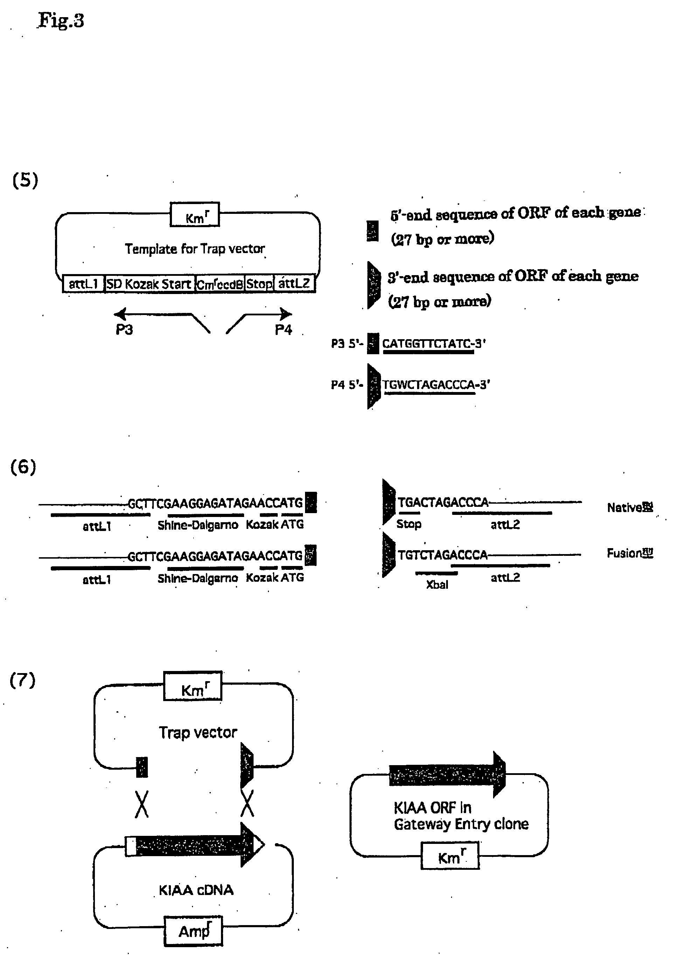

The base sequences of the first primer (P3) and second primer (P4) used for the above PCR were as follows, and the constructions thereof are shown in Fig. 3(5).
Primer P3 (Sequence No.1): 5'-gtt ttg gaa atg tgt gac gag gca acc agc ttc aca tcc ccc atg gtt cta tc-3' (the underlined part shows an antisense sequence of the 5'-teminal sequence of the myosin light chain kinase gene contained in P3)
Primer sequence P4 (sequence No. 2): 5'-acg atg gag gaa ggt gaa ggg gaa ggg gaa gag gaa gaa gag tgw cta gac cca-3' (the underlined part shows an antisense sequence of the 3'-terminal sequence of the myosin light chain kinase gene contained in P4)

### (7) Preparation of pENTRhg00295N1

pENTRhg00295N1 (an entry clone) was obtained by subcloning the ORF in an entry vector of the Gateway system from a clone hg00295 containing the myosin light chain kinase gene by means of a homologous recombination reaction in the Escherichia coli cells. 20 µl of Escherichia coli JC8679 strain (obtained from Health Science Research Resources Bank, accession No. YG-HT017) was transformed with a Notl digest product (5 µg/2 µl sterilized water) of the clone hg00295 and 500 ng of the trap vector for hg00295 prepared above by an electroporation method (E. coli pulser manufactured by BioRad Co.; 1.67 kv, 5.0 msec, 1 mm cuvette). SOC (200 µl) was added to the solution and, after cultivating at 37°C for 1 hour, 100 µl of the culture medium was applied on a plate containing 50 µl/ml kanamycin to obtain 300 or more regeneration colonies. The colonies. (10 colonies) were seeded in 2 ml of LB liquid culture medium containing 60 µg/ml of kanamycin, and a plasmid was prepared using a Concert Rapid Plasmid purification kit manufactured by invitrogen Co. after cultivation with shaking (37°C, O/N). The results of Xball digestion showed that 4 colonies were digested but 6 colonies were not with Xball. It was shown by checking boundary regions between the terminal of the ORF and each vector of these clones that the clone digested with Xball was a fusion type while the clone not digested with Xball was a native type. One of the six native clones was named as pENTRhg 00295N1 (279 ng/µl).

### Preparation of pT7DESThg 00295N1

The ORF part of hg00295 of pENTRhg 00295N1 was transferred to pT7DEST (prepared by converting the Nrul-Ncol site of pET-DEST42 (manufactured by invitrogen Co.) into Nrul-Ncol site of pDEST24 (manufactured by Invitrogen Co.)) as a destination vector. The LR reaction was performed In a 20-µl system (1 µl of pENTRhg 00295N1 (279 ng/µl), 2 µl of EcoR digest (20 ng/µl of pT7DEST, 4 µl of 5 × LR buffer, 9 µl of sterilized water and 4 µl of LR clonase mix) at a room temperature for 1 hour. The solution was warmed at 37°C for 10 minutes after adding 2 µl of protein kinase K. After mixing 1 µl of the LR reaction solution and 50 µl of competent cells (UBRARY EFFICIENCY DH5α, manufactured by invitrogen Co.) for the DH5α calcium method, the mixed solution was allowed to stand on ice for 30 minutes. After applying heat shock treatment at 42°C for 30 seconds, the solution was cooled on ice for 2 minutes. After the addition of 450 µl of SOC, the cells were cultivated at 37°C for 1 hour, 200 µl of the culture medium was applied on a plate containing 50 µg/ml of ampicillin so that 300 or more regeneration colonles were obtained. The colonies were seeded In 2 ml of the LB liquid culture medium containing 50 µg/ml of ampicillin and, after cultivation with shaking (37°C, O/N), 75 µl (120 ng/µl) of pT7DESThg 00295N1 as an expression clone was obtained using a Concert Rapid Plasmid purification kit manufactured by BRL Co. The plasmid was precipitated with EtOH after treating with phenol/chloroform/isoamyl alcohol (PCl), and the precipitate was dissolved in 10 µl of nuclease-free water (208 ng/µl).

### Confirmation of the In vitro transcription/translation reaction product of pT7DESThg 00295N1

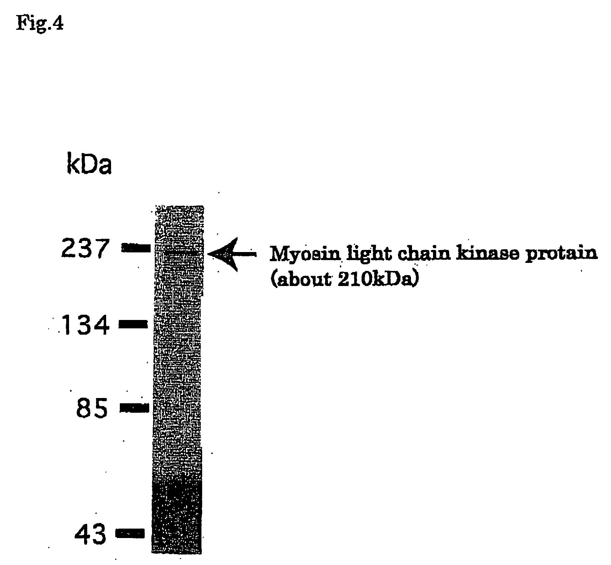

The product of pT7DESThg 00295N1 was confirmed by an In vitro transcription/translation reaction using TNT Quick Coupled Transcription/Translation Systems manufactured by Promega Co. The transcription/translation reaction was carried out in a 50-µl system (40 µl of TNT Quick Master Mix, 1 µl of 1 mM methionine, 5 µl of pT7DESThg 00295N1 (208 ng/µl), 2 µl of Fluoro Tect (manufactured by Promega Co.), 2 µl of nuclease-free water) at 30°C for 90 minutes. Subsequently, the in vitro transcription/translation product was confirmed by Laemmli SDS-PAGE. An electrophoresis sample prepared by mixing 2 µl of the transcription/transtation product, 3 µl of a phosphate buffer solution, 1 µl of 6 × sample buffer and 0.1 µl of mercaptoethanol (14.4 M) was denatured in boiling water for 3 minutes, and subjected to electrophoresis (40 mA, 1 hour) on a 7.5% SDS-PAGE mini-gel (8.5 cm × 8.5 cm). Prestained standards (5 µl: "Kaleidscope" manufactured by BioRad Co.) were used as markers for electrophoresis. The gel was placed on a non-fluorescent glass plate, and a band at about 210 kDa was detected (Fig. 4) using Fluorlmager 595 (manufactured by Molecular Dynamics Co., detection condition: PMT voltage 700, excitation filter 488 nm, emission filter 530 DF30). The result was in good agreement with 210,130Da of a calculated molecular weight of the myosin light chain kinase protein.

### Example 2

An additional 10 genes were cloned as in Example 1 by the method according to the present invention. The results in Table 1 show that an accurate, quick and simple cloning of ORFs comprising a very large number of bases In the range of 7,000 to 10,000 or more was successful.

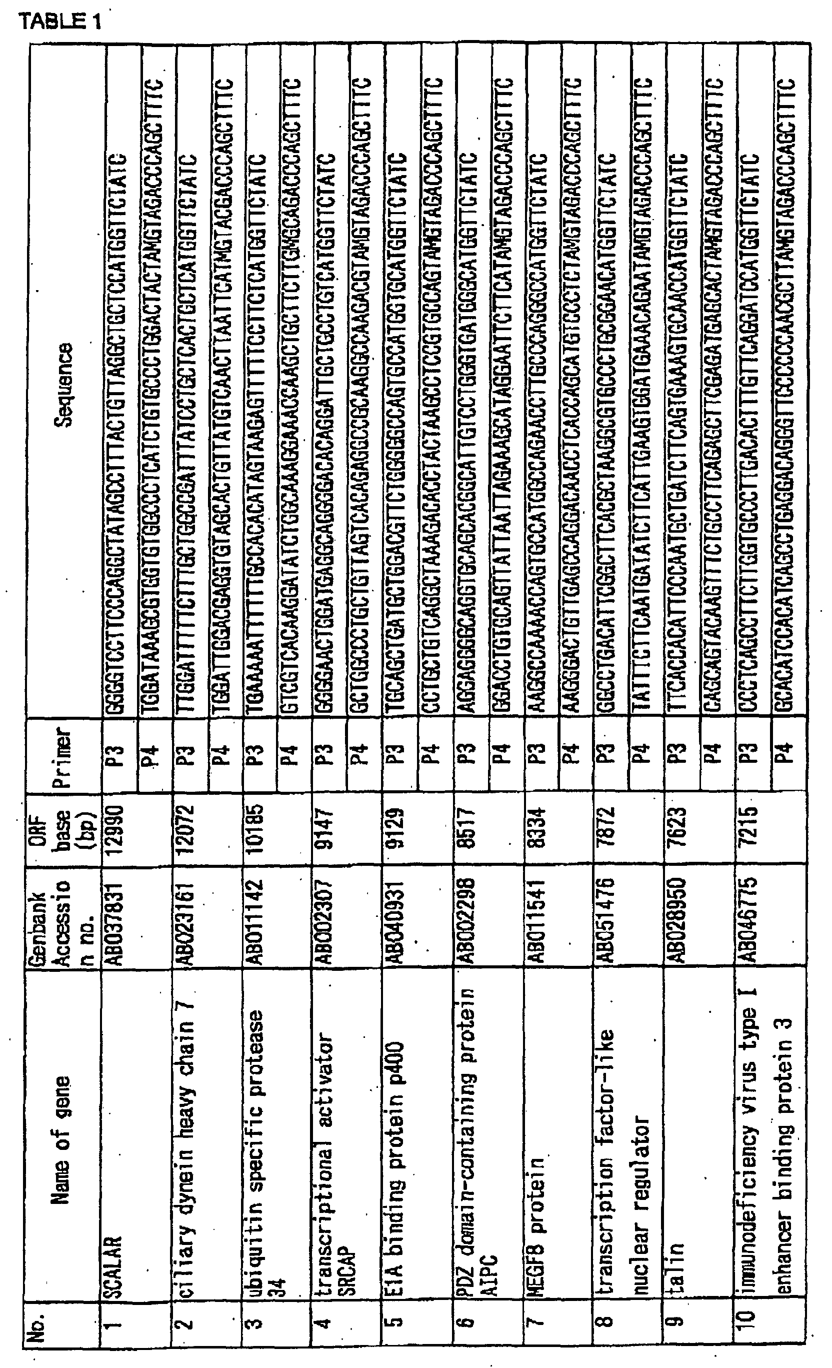

### Example 3

### Expression in culture cells and expression of full-length protein

Trap vectors for subcloning the ORF of OL-protocadherin (GenBank accession No. AB037821) and sodium channel β-subunit (GenBank accession No. AB032984) were prepared using PCR In the same manner as in Example 1 using the template vector for the trap vector and the primers shown in Table 2 below. Each ORF was sub-cloned from the clone (hk08136) and clone (hj00081) containing the gene described above, respectively, in the entry vector of the Gateway system to obtain pENTRA1400F1 and pENTRA1158F1 as entry colnes.

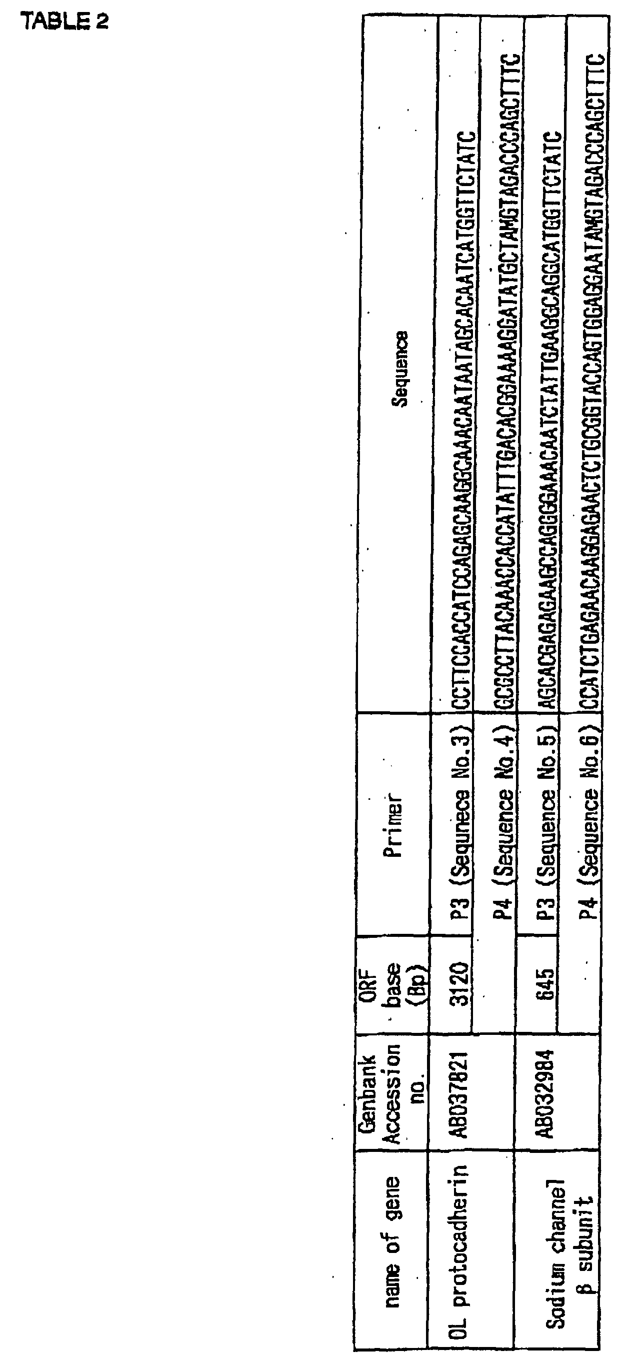

The ORF of each gene contained in each entry clone above was transferred to pcDNADEST47 (manufactured by Invltrogen, Carlsbad, CA; this plasmid has a CMV promoter and produces proteins In which GFP is fused at the C-terminal of the desired protein) by the LR reaction. Opti-MEM (250 µl, manufactured by Invitrogen Co.) was added to 20 µl (4 µg) of a diluted solution of the plasmid. A cation-lipid transfection reagent (10 µl, Lipofectamine 2000 manufactured by Invitrogen C., Carisbad, CA. USA) was mixed with 250 µl of Opti-MEM. The plasmid solution was mixed with the transfection reagent solution and, after allowing to stand at a room temperature for 30 minutes, the mixed solution was sprayed on 80 to 90% confluent Flp in 293 cells (manufactured by Invitrogen Co.) in a 6-well plate for transfection.

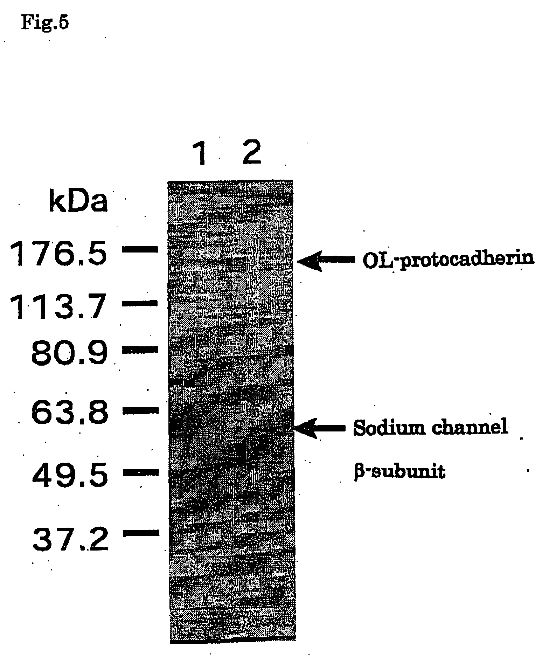

Three days after transfection, the cells were lysed by adding 100 µl of 4 × sample buffer (Ausbel, F., Brent, R., Kingston, R., Moore, D., Seidman, F., Smith, J. and Struhl, K., 1987, Current protocol in Molecular Biology, New York, JOHN WILEY & SONS), and the cytosol was retrieved using a policeman. It was confirmed that the viscosity of the cytosol was reduced (fragmentation of DNA) by applying an ultrasonic wave (VP-5S manufactured by TAITEC Co., output control 10, 20 seconds). Then, the sample was analyzed by SDS-PAGE using an 8% acrylamide gel, The gel after electrophoresis was immersed In a blotting buffer (25 mM Tris, 192 mM glycine, 20% methanol) for 15 minutes, and transferred onto a PVDF membrane (manufactured by Pall Co., East Hills, NY, USA) at a constant current of 100 mA for 1 hour (BE-300 manufactured by BtOCRAFT Co., Tokyo, Japan). The membrane was immersed In TBST (0.05% Tween 20 in TBS (20 mM Tris-Cl, 150 mM NaCl)) for 10 minutes, then in a blocking solution (10% BSA in TBS) for 1 hour. After being Immersed in a primary antibody solution (GFP anti-serum, manufactured by Invitrogen Co.) diluted 5,000 times with the blocking solution, the membrane was washed 4 times with TBST for 10 minutes and TBS for 5 minutes. Then, the membrane was Immersed in a secondary antibody solution (alkaline phosphatase-linked anti-rabbit IgG antibody, manufactured by CAPPEL Co., Aurora, Ohio, USA) diluted 4,000 times with the blocking solution for 1 hour. After washing the membrane four times with TBST for 10 minutes and TBS for 5 minutes, the membrane was subjected to a coloring reaction for 60 minutes by adding a substrate solution (Western blue, manufactured by Promega Co., Madison, Wl, USA). The results are shown In Flg. 5.

While a fusion protein of OL protocadherln (GenBank accession No. AB0378) and GFP migrated in lane 1, and a fusion protein of sodium channel β-subunit (GenBank accession No. AB032984) and GFP migrated in lane 2 In the electrophoresis diagram In Fig. 5, positive bands at about 140kDa and about 50 kDa are confirmed In lane 1 and lane 2, respectively. Since these bands are in agreement with predicted molecular weights of 141,188 Da and 53,146 Da, respectively, the whole length fusion proteins corresponding to the fusion of respective proteins and GFP are assumed to be expressed.

### Industrial Applicability

The invention enables the accurate, quick and simple cloning of a target ORF, or a long-chain target ORF having several thousand or more bp, which is, for example, a cDNA contained in a vector.

Since the linear trap vector prepared in the present invention comprises a 5'-end sequence and 3'-end sequence of the target ORF at each end thereof, respectively, a cloning vector incorporating only the target ORF in the trap vector can be prepared by a homologous recombination reaction between the trap vector and target ORF. Consequently, adverse effects caused by untranslated regions (UTRs) at the upstream and downstream sites of the ORF region may be avoided.

Furthermore, DNAs containing the target ORF are not required to be previously purified in the cloning method of the present invention. in addition, since the target ORF is amplified by taking advantage of a replication mechanism In Escherichia coil cells, any mutations observed in conventional PCR amplification will not occur.

The cloning vector of the present invention, which is prepared using a template vector for the trap vector containing the first primer-binding sequence and the second primer-binding sequence between attL1 and attL2 prepared by the BP reaction in a site-specific recombination system of λ-phage, is able to function as an entry clone in Gateway technology. Expression clones linked to promoters and tags for various desired expression systems can be obtained with high throughput while maintaining the target ORF by homologous recombination (the LR reaction) between the entry clone and various destination vectors In this Gateway technology.

## Claims

1. A template vector for a trap vector comprising a replication origin, a first drug tolerance gene, a first primer-binding sequence, a second primer-binding sequence, and a suicide gene inserted between the first and second primer-binding sequences.

2. The template vector for a trap vector according to Claim 1, wherein the first primer-binding sequence comprises at least a part of a Kozak consensus sequence, and the second primer-binding sequence comprises a TGN or TAN sequence, N denoting G, A, T or C.

3. The template vector for a trap vector according to Claim 1 or 2 comprising a Shine-Dalgarno sequence upstream of the 5'-end of the consensus sequence.

4. The template vector for a trap vector according to any one of Claims 1 to 3 further comprising a restriction enzyme site containing the TGN or TAN sequence.

5. The template vector for a trap vector according to any one of Claims 1 to 4, wherein the first primer-binding sequence and second primer-binding sequence comprise at least five bases.

6. The template vector for a trap vector according to any one of Claims 1 to 5, wherein a second drug tolerance gene is further inserted between the first and second primer-binding sequences.

7. The template vector for a trap vector according to any one of the Claims 1 to 6 comprising the first and second primer-binding sequences between attL1 and attL2.

8. The template vector for a trap vector according to Claim 7 prepared using a BP reaction in a site-specific recombination system of λ-phage.

9. PCR primers for forming a trap vector comprising a first primer comprising an antisense sequence of the 5'-end sequence of a target open reading frame (ORF) and an antisense sequence of the first primer-binding sequence linked to the 3'-side thereof, and a second primer comprising a sense sequence of the 3'-end sequence of the target ORF and a sense sequence of the second primer-binding sequence linked to the 3'-side thereof.

10. The PCR primers for forming a trap vector according to Claim 9, wherein the 5'-end sequence and 3'-end sequence of the target ORF each comprises at least 10 bases.

11. A kit for forming a trap vector comprising the template vector for a trap vector according to Claims 1 to 8 and the PCR primers according to Claim 9 or 10.

12. A method for forming a linear trap vector by PCR using the kit for forming the trap vector according to Claim 11.

13. A method for forming a cloning vector containing the target ORF in the trap vector by a homologous recombination reaction between the trap vector obtained by the method according to Claim 12 and the target ORF.

14. The method according to Claim 13, wherein a RecE/RecT enzyme is used In the homologous recombination reaction.

15. The method according to Claim 13 or 14, wherein the target ORF is cDNA.

16. The method according to any one of Claims 13 to 16, wherein the target ORF is contained in a vector.

17. The method according to any one of Claims 13 to 16, wherein the target ORF contains genes encoding genetic information.

18. The method according to any one of Claims 14 to 17, wherein bacteria are co-transformed with the trap vector and the vector containing the target ORF to effect homologous recombination in the bacteria.

19. The method according to Claim 18, wherein a regenerated colony is obtained by cultivating co-transformed bacteria for one to several hours.

20. The method according to Claim 18 or 19, wherein the bacteria are Escherichia coli having a high homologous recombination activity.

21. A cloning vector obtained by the method according to any one of Claims 13 to 20.

22. The cloning vector according to Claim 21 functioning as an entry clone in Gateway technology.

23. A cloning method of the target ORF comprising a step of amplifying the cloning vector according to Claim 21 or 22.

24. A gene encoded in the target ORF cloned by the method according to Claim 23.


SEQUENCE LISTING

<110> KAZUSA DNA RESEARCH INSTITUTE FOUNDATION
<120> Method for Efficient Homologous Recombination of ORF Encoded in
cDNA
<130> PCT-AB03044

<150> JP 2002-357566
<151> 2002-12-10

<160> 6

<210> 1
<211> 53
<212> DNA
<213> Artificial
<220>
<221> primer_bind
<223> Primer P3
<400> 1
gttttggaaa tgtgtgacga ggcaaccagc ttcacatccc ccatggttct atc    53

<210> 2
<211> 54
<212> DNA
<213> Artificial
<220>
<221> primer_bind
<223> Primer P4
<400> 2
acgatggagg aaggtgaagg ggaaggggaa gaggaagaag agtgwctaga ccca    54

<210> 3
<211> 53
<212> DNA
<213> Artificial
<220>
<221> primer_bind
<223> Primer P3
<400> 3
ccttccacca tccagagcaa ggcaaacaat aatagcacaa tcatggttct atc    53

<210> 4
<211> 60
<212> DNA

<213> Artificial
<220>
<221> primer_bind
<223> Primer P4
<400> 4
gcgccttaca aaccaccata tttgacacgg aaaaggatat gctamgtaga cccagctttc    60

<210> 5
<211> 53
<212> DNA
<213> Artificial
<220>
<221> primer_bind
<223> Primer P3
<400> 5
agcacgagag aagccagggg aaacaatcta ttgaaggcag gcatggttct atc    53

<210> 6
<211> 60
<212> DNA
<213> Artificial
<220>
<221> primer_bind
<223> Primer P4
<400> 6
ccatctgaga acaaggagaa ctctgcggta ccagtggagg aatamgtaga cccagctttc    60